# EUROPEAN PATENT SPECIFICATION

(11) **EP 0 812 859 B1**
(45) Date of publication and mention of the grant of the patent: **13.02.2002**
(21) Application number: 96943313.5
(22) Date of filing: 26.12.1996
(51) Int. Cl.: C07K 16/36, C12P 21/08, C12N 15/06, C12N 5/18, G01N 33/53, G01N 33/577, C07K 16/40, C07K 16/38

(54) **ANTIFACTOR Xa-TISSUE FACTOR PATHWAY INHIBITOR COMPLEX MONOCLONAL ANTIBODY AND USE OF THE SAME**
MONOKLONALER ANTIKÖRPER GEGEN DEN KOMPLEX AUS FAKTOR XA UND DEM INHIBITOR FÜR DEN GEWEBEFAKTOR (XA. TFPI)
ANTICORPS MONOCLONAL, ANTI COMPLEXE "Xa . TFPI" ET SON UTILISATION

(30) Priority: 26.12.1995 JP 33922995
(43) Date of publication of application: 17.12.1997
(73) Proprietor: IATRON LABORATORIES, INC., Chiyoda-ku Tokyo 101 (JP); Juridical Foundation, The Chemo-Sero-Therapeutic Research Institute, Kumamoto-shi, Kumamoto 860-8568 (JP)
(72) Inventor: KOHNO, Isao Iatron Laboratories, Inc., Tokyo 101 (JP); ITOH, Yumiko Iatron Laboratories, Inc., Tokyo 101 (JP); KUMETA, Kosuke, Tamana-gun, Kumamoto 869-03 (JP); KAMIKUBO, Yuichi, Kumamoto-shi, Kumamoto 860 (US); SOE, Gilbu Iatron Laboratories, Inc., Tokyo 101 (JP)
(74) Representative: Cohausz & Florack
(86) International application number: JP9603832
(87) International publication number: WO9723509

(56) References cited:
- GIRARD T J ET AL: "FUNCTIONAL SIGNIFICANCE OF THE KUNITZ-TYPE INHIBITORY DOMAINS OF LIPOPROTEIN-ASSOCIATED COAGULATION INHIBITOR" NATURE,GB,MACMILLAN JOURNALS LTD. LONDON, vol. 338, April 1989 (1989-04), pages 518-520, XP000857349 ISSN: 0028-0836
- BROZE JR G J ET AL: "REGULATION OF COAGULATION BY A MULTIVALENT KUNITZ-TYPE INHIBITOR" BIOCHEMISTRY,US,AMERICAN CHEMICAL SOCIETY. EASTON, PA, vol. 29, no. 33, 21 August 1990 (1990-08-21), pages 7539-7546, XP000268583 ISSN: 0006-2960
- GIRARD T J ET AL: "INHIBITION OF FACTOR VIIA-TISSUE FACTOR COAGULATION ACTIVITY BY A HYBRID PROTEIN" SCIENCE,US,AMERICAN ASSOCIATION FOR THE ADVANCEMENT OF SCIENCE,, vol. 248, no. 4961, 15 June 1990 (1990-06-15), pages 1421-1424, XP000253643 ISSN: 0036-8075
- GOUIN-THIBAULT I ET AL: "PROBABLE REGULATION OF FACTOR VIIa-TISSUE FACTOR AND PROTHROMBINASE BY FACTOR Xa-TFPI AND TFPI IN VIVO" BRITISH JOURNAL OF HAEMATOLOGY, vol. 95, no. 4, pages 738--746, XP000872775
- J. BIOCHEM., Vol. 118, No. 1, (1995), "An Anti-Tissue Factor Pathway Inhibitor Monoclonal Antibody ...", pages 178-182.
- J. BIOCHEM., Vol. 115, No. 4, (1994), "Amino Acid Sequence and Inhibitory Activity ...", pages 708-714.

## Description

### FIELD OF THE INVENTION

The present invention relates to an anti-factor Xa-tissue factor pathway inhibitor complex monoclonal antibody, a hybridoma secreting said monoclonal antibody, and a method for immunologically measuring said anti-factor Xa-tissue factor pathway inhibitor complex.

### RELATED ART

It is important to measure factor Xa (also referred to as Xa hereinafter) converting prothrombin to thrombin in blood in the clinical field, in order to examine coagulation sthenic conditions, such as disseminated intravascular coagulation (DIC) syndrome and thrombosis. However, it is very difficult to measure produced Xa. This is because, immediately after it is produced, it binds to a tissue factor pathway inhibitor (also referred to as TFPI) to form a factor Xa-tissue factor pathway inhibitor complex (also referred to as Xa-TFPI).

Thus, the simplest method for measuring the amount of Xa produced in blood, is to quantify the Xa-TFPI which reflects indirectly the amount of Xa produced.

To date, to measure Xa-TFPI, a sandwich EIA (Enzyme Immuno Assay) combining two different antibodies has been used: one is a polyclonal or monoclonal antibody to factor X (also referred to as X) and Xa, and the other is a polyclonal or monoclonal antibody to TFPI.

However, measuring Xa-TFPI with accuracy has been difficult in the prior art method, because the measurements may be interfered by any free X or free TFPI which is present in the sample.

### DISCLOSURE OF THE INVENTION

The inventors carried out intensive experiments to solve the defect in the prior art sandwich EIA used to quantify Xa-TFPI which is present in blood, the measurements in the EIA being interfered with by any free X or free TFPI which is present in the sample. As a result, the inventors found out that a monoclonal antibody specific to Xa-TFPI can be obtained by binding Xa to TFPI to form a complex, by producing a hybridoma yielding and secreting the monoclonal antibody which recognizes the newly occurred antigen determinant, and by separating and purifying the culture of the hybridoma. Thus, the present invention also provides a method for measuring Xa-TFPI with simplicity, accuracy and reproducibility by utilizing the newly found monoclonal antibody.

That is, the present invention relates to a monoclonal antibody which reacts with Xa-TFPI with specificity, but does not react with free factor X, with free factor Xa and with free TFPI.

Further, the present invention relates to a hybridoma or the cell strain derived from it, which secretes monoclonal antibody that is produced by cell fusion of a spleen cell of a mammal immunized with Xa-TFPI with a myeloma cell of a mammal and that reacts with Xa-TFPI with specificity, but does not react with free factor X, with free factor Xa and with free TFPI.

Furthermore, the present invention relates to a method for immunologically measuring Xa-TFPI, comprising using the monoclonal antibody.

### THE BRIEF DESCRIPTION OF THE DRAWINGS

Fig. 1 is a graphical representation showing the binding forces of anti-Xa-TFPI monoclonal antibody (XT-1) to Xa, TFPI and Xa-TFPI, which were obtained by an enzyme immuno assay comprising determining the reactivities of XT-1 in ascitic fluid with the antigens directly coated onto 96-well micro-titer plates.

Fig. 2 is a graphical representation showing a calibration curve composed of reaction velocities of latex agglutinations, determined in LPIA-100, and concentrations of Xa-TFPI.

Fig. 3 is a graphical representation showing the results of sandwich enzyme immuno assay on Xa-TFPI, etc., wherein the anti-TFPI monoclonal antibody was used as the first antibody, and the anti-Xa-TFPI monoclonal antibody (XT-1) was used as the enzyme labeled second antibody.

### PREFERRED EMBODIMENT FOR CARRYING OUT THE INVENTION

Hereinafter, the present invention will be illustrated in detail.

An anti-Xa-TFPI monoclonal antibody may be prepared by culturing each of the novel mice hybridoma in culture medium or the ascitic fluid of the mice.

The mice hybridoma used in the present invention may be generally produced by cell fusion of a spleen cell of a mammal immunized with Xa-TFPI with a mice myeloma cell, according to the basic cell fusion method by Köhler and Milistein [Nature, vol. 256, p. 495 (1975)]. The details of the method will be illustrated in Examples described below.

The medium in which the hybridoma is cultured may be one that is suitable for culturing the hybridoma, preferably, Dulbecco's Modified Eagle's minimum essential medium (referred to as DME) including fetal bovine serum, L-glutamine, L-pyruvic acid and antibiotics (penicillin G and streptomycin) may be used.

The anti-Xa-TFPI monoclonal antibody may be isolated and purified from the culture or the mice ascitic fluid thus prepared, according to the methods generally used in isolation and purification of proteins.

Such methods may be salting out, ion exchange column chromatography with an ion exchange cellulose, molecular sieve column chromatography with a molecular sieve gel, affinity column chromatography with Protein A binding polysaccharides, dialysis, lyophilyzation, or the like.

The anti-Xa-TFPI monoclonal antibody thus prepared may have the ability to bind only Xa-TFPI, but not to bind free X, free Xa and free TFPI, and may be useful as a reagent used to immunologically quantify Xa-TFPI. The anti-Xa-TFPI monoclonal antibody of the present invention (for example, XT-1 obtainable in Examples described below) may be used as a reagent in enzyme immuno assay (EIA), fluoro immuno assay (FIA), luminescent immuno assay (LIA) or radioactive immuno assay (RIA), or the like. An example of EIA may be one-step sandwich enzyme immuno assay using micro-plates or plastic tubes.

Although the embodiment of EIA may be as in Example 6 described below, it may be in general carried out as follows:

An anti-Xa-TFPI monoclonal antibody may be used; Firstly, as the first antibody, the anti-Xa-TFPI monoclonal antibody of the present invention (XT-1) is immobilized in the wells of micro-plates or in plastic tubes. Secondly, a sample including Xa-TFPI and a solution of the anti-TFPI antibody labeled with an enzyme as the second antibody is placed into the wells or plastic tubes. Then, the wells or plastic tubes are washed after allowing to leave for about 30 minutes, to which a solution of the enzymal substrate is added, allowing to the enzymal reaction for about 30 minutes, and after completion of the reaction, the amounts of Xa-TFPI in the sample are quantified by colorimetric analysis.

Alternately, one may quantify Xa-TFPI in blood using an anti-TFPI monoclonal antibody [for example, the anti-TFPI monoclonal antibody produced by the hybridoma HTFPI-K9 acquired in The Chemo-Sero-Therapeutic Research Institute (deposit no.: FERM-P-14467)] as the first antibody, and the anti-Xa-TFPI monoclonal antibody of the present invention (XT-1) labeled with an enzyme, etc. as the second antibody, according to the procedure as described above.

Further, the monoclonal antibody of the present invention may be used in a latex agglutination immuno assay as described below, wherein one may quantify Xa-TFPI in a sample by contacting the analyte to the latex to which the monoclonal antibody of the present invention is immobilized, and by determining the presence of the resultant aggregate.

### EXPERIMENTS

The present invention will be illustrated in Examples hereinafter in more detail.

### Example 1: Preparation of Xa-TFPI

The Xa-TFPI was prepared as in the following procedure. 5 mg of purified Xa (10 ml of solution containing 0.5 mg/ml of Xa) and 15 mg of TFPI (3 ml of solution containing 5 mg/ml of TFPI) were mixed and allowed to react for an hour at 37°C, and then the reaction was terminated by adding 13 ml of a solution contains 1M diisopropyl fluorophosphoric acid to the reaction solution. The mixture was charged into a haparin-Sepharose column (column volume: 100 ml) previously equilibrated with 20 mM Tris-HCl buffer containing 0.1M NaCl (pH 7.4).

After washing the column with 400 ml of the buffer, it was eluted with the linear gradient between 20 mM tris-HCl buffer containing 0.1M NaCl (pH 7.4) and 20 mM Tris-HCl buffer containing 0.5M NaCl (pH 7.4). The eluent was collected in each of 5 ml of fraction. Whether or not a fraction contained the complex, was determined by polyacrylamide gel electrophoresis. The fraction nos. 61 - 120 containing Xa-TFPI were collected and dialyzed against 20 mM Tris-HCl buffer containing 50 mM NaCl.

Then, the dialysate was charged into DEAE-Sephacel column (column volume: 50 ml, Parmacia) previously equilibrated with 20 mM Tris-HCl buffer containing 50 mM NaCl. After washing the column with about 200 ml of the buffer, it was eluted with a linear gradient of 20 mM Tris-HCl buffer containing 0.05M NaCl. The eluent was collected in each of 2 ml of fraction. The fractions containing Xa-TFPI were collected in the same manner, and concentrated by ultrafiltration to give 5 ml of Xa-TFPI (A280 nm = 1.0). The resultant Xa-TFPI solution was used as an immunogen and an antigen in ELISA, which was used for selecting the hybridoma producing anti-Xa-TFPI antibody.

### Example 2

### (a) Preparation of Immunized Spleen Cell:

The Xa-TFPI immunogen solution (A280 = 0.1) was mixed with the equivalent volume of Freund's complete adjuvant to emulsion, and an immunization was carried out by administering 200 µl of the mixture intraperitoneally to mice (the first immunization). After the period of 30 days, the solution was administered intraperitoneally to the mice in the same manner (the second immunization). After the period of 21 days from the second immunization, Xa-TFPI immunogen solution (A280 = 0.1) was diluted with the equivalent amount of saline, and 200 µl of the diluent was administered intravenously to the mice (the final immunization). After the period of 3 days from the final immunization, spleen cells were excised from the mice and then used for cell fusion.

### (b) Cell Fusion:

The spleen excised aseptically was placed into a Petri dish in which 5 ml of DME medium containing 10 to 15% fetal bovine serum was placed. The spleen was then perfused with about 15 ml of DME medium containing 10 to 15% fetal bovine serum to dissolve it into the cells, and then the suspension of the spleen cells was passed through a nylon mesh. These spleen cells were collected into a 50 ml centrifuge tube and centrifuged at 500 xg for 10 minutes. To the resultant pellet was added 3 to 5 ml of hemolyzing solution (155 mM NH₄Cl, 10 mM KHCO₃, 1 mM Na₂EDTA, pH 7.0) to form the suspension. Allowing to stand at 0°C for 5 to 10 minutes resulted in the disruption of the erythrocytes in the suspension. To the suspension was added 10 to 20 ml of DME medium containing 10 to 15% fetal bovine serum and then it was centrifuged. The resultant pellet was washed with DME medium through centrifugation and the number of the live spleen cells was determined.

1 × 10⁸ of spleen cells were added to about 2 × 10⁷ of mouse myeloma cells SP2/0-Ag14 which were precultured, they were mixed well in DME medium, and the mixture was centrifuged (500 xg, 10 minutes). The supernatant was aspirated, the pellet was well resolved, 0.5 ml of 40% polyethyleneglycol 4000 solution kept at 38°C was dropped to it, and the polyethyleneglycol solution and the pellet of cells were mixed by rotating gently the centrifuge tube by a hand for 1 minute. Then, 1 ml of DME medium kept at 38°C was added every 30 seconds to the tube, which was then rotated gently.

The above procedure was repeated 10 times, then 20 to 30 ml of DME medium containing 10 to 15% fetal bovine serum was added to the tube, which was then centrifuged (500 xg, 10 minutes). After removing the supernatant, the pellet of cells were washed 2 times with HAT medium (4 × 10⁻⁷M aminopterin, 1.6 × 10⁻⁵M thymidine, and 1 × 10⁻⁴M hypoxantin in DME medium) containing 10 to 15% fetal bovine serum through centrifugation, and then the pellet was suspended in 40 ml of the HAT medium. 200 µl of the suspension of the cells was dispensed into each of well in a 96-wells cell culture plate, and the wells were subject to start culturing a carbon dioxide fermenter with 5% carbon dioxide.

During the period of the culture, 100 µl of the medium in each well was removed every 2 or 3 days and 100 µl of fresh HAT medium was added to it, and thereby the hybridomas growing in HAT medium were selected. After the cultivation period of nearly 8 days, the culture medium was replaced with HT medium (1.6 × 10⁻⁵M thymidine, and 1 × 10⁻⁴M hypoxantin in DME) containing 10 to 15% fetal bovine serum, the propagation of the hybridoma was observed, and the hybridomas producing an anti-Xa-TFPI antibody were screened by the ELISA mentioned below.

### (c) Establishment of Hybridoma

The presence or absence of antibodies produced in the supernatant of the hybridoma culture was determined by ELISA. 50 µl of the aforementioned purified Xa-TFPI solution (A280 = 0.05, diluted with saline) was dispensed in each of the wells in a 96-well ELISA plate (Immulon, Nippon Dynatec Co., Ltd.), and allow to stand at 25°C for 2 hours. Then, the wells were washed 3 times with 0.05% of Tween 20 in saline, the supernatant of the culture was then added to each of the wells, and the wells were allowed to react at 25°C for 1 hour.

50 µl of solution of anti-mice antibody bound to peroxidase (Daco, Co., Ltd., Denmark) diluted 200 times with 0.05% of Tween 20 in saline, was added to each of the wells. After the completion of the reaction, the wells were washed 3 times with 0.05% Tween 20 in saline, 200 µl of the solution containing 0.5 mM aminoantipyrine, 10 mM phenol and 0.005% hydrogen peroxide was added to each of the wells, the wells were allowed to react at 25°C for 30 minutes, and the optical density at 490 nm of each of the wells was determined. The results revealed that in 23 wells out of 192 wells production of antibodies was detected.

Whether or not anti-Xa-TFPI antibody detected in the supernatant of the culture by the ELISA reacts with TFPI and Xa, was determined using a 96-well ELISA plate to which TFPI and Xa were bound in the same manner as described above. The results demonstrated that among 23 wells in which the supernatants of the culture reacted with Xa-TFPI, the supernatants of culture in 18 wells also reacted with TFPI, but did not react with Xa at all.

The hybridoma in one well, which did not react TFPI and Xa, but reacted with specificity only with Xa-TFPI, was transferred to a 24-well plate, and cultured in HT medium containing 10 to 15% fetal bovine serum for 4 or 5 days.

Subsequently, the presence or absence of production of the anti-Xa-TFPI specific antibody (hereinafter referred to as anti-Xa-TFPI antibody for short) was confirmed again by ELISA, and then the hybridomas were cloned by limiting dilution analysis. The limiting dilution analysis was as follows: 100 µl of the cell suspension in which the hybridomas were diluted to 5 cell/ml in HT medium, was dispensed in each of the wells in a 96-well plate in which peritoneal cells of a normal BALB/C mice had been dispensed at 2 × 10⁴ cells per well. After about 10 days, clones of hybridomas which produced the anti-Xa-TFPI antibody were screened by ELISA. The results demonstrated that each of the hybridomas resulted in 20 to 40 clones which produce the antibody.

Among the clones, clones which were proliferative, had high antibody secretion-ability, and were stable, were selected, and cloned again in the same manner as described above to establish the hybridoma XT-1 which produces anti-Xa-TFPI specific antibody.

The hybridoma XT-1 was deposited at the National Institute of Bioscience and Human-Technology (NIBH), Agency of Industrial Science and Technology, under the deposit no. FERM P-15357, on December 20, 1995, and transferred to the international depositories under the deposit no. FERM BP-5776 on December 20, 1996, according to the Budapest Treaty.

### Example 3: Production of Monoclonal Antibody

### In Vitro method

The mice hybridoma XT-1 was cultured in DME medium containing 15% fetal bovine serum at 37°C in an atmosphere of 5% carbon dioxide for 72 to 96 hours.. The culture was centrifuged (10,000 xg, 10 minutes), and then to the supernatant was gradually added ammonium sulfate to a final concentration of 50%. The mixture was stirred in an ice bath for 30 minutes, allowed to stand for 60 minutes, centrifuged (10,000 xg, 10 minutes), the resultant residue was dissolved in small volume of 10 mM phosphate buffer (pH 8.0), and then the solution was dialyzed against 1,000 times volume of 10 mM phosphate buffer.

The dialyzed solution was charged into a column packed with DEAE-cellulose which had been equilibrated with 10 mM phosphate buffer. The monoclonal antibody was eluted with a gradient eluent between 10 mM phosphate buffer (pH 8.0) and 10 mM phosphate buffer (pH 8.0) containing 0.2M NaCl. The eluted monoclonal antibody was concentrated by ultrafiltration, and the condensate was dialyzed against 0.1M phosphate buffer (pH 8.0). To remove bovine serum IgG, the dialysate was passed through a column packed with goat anti-bovine serum IgG-Sepharose 4B. The passed liquid was charged into a column packed with Protein A-Sepharose 4B which had been equilibrated with 0.1M phosphate buffer (pH 8.0). The column was eluted with the buffer at pH 3.5 to give a solution of purified anti-Xa-TFPI antibody XT-1.

### In Vivo method

0.5 ml of pristane (2,6,10,14-tetramethylpentadecane) was administered intraperitoneally to 10 to 12 weeks old BALB/C mice, after 14 to 20 days, the mice was inoculated intraperitoneally with the hybridomas XT-1 grown in vitro at 2 × 10⁶/a mice.

About 10 to 15 ml of ascitic fluid was obtained from one mice. The concentrations of antibodies were 2 to 10 mg/ml. The monoclonal antibodies in the ascitic fluid were purified in the same manner as described above in the In Vitro Method section, except that the passing procedure with the column packed with goat anti-bovine serum IgG-Sepharose 4B was omitted.

### Example 4: Identification of Immunoglobulin Class and Specificity of Monoclonal Antibody

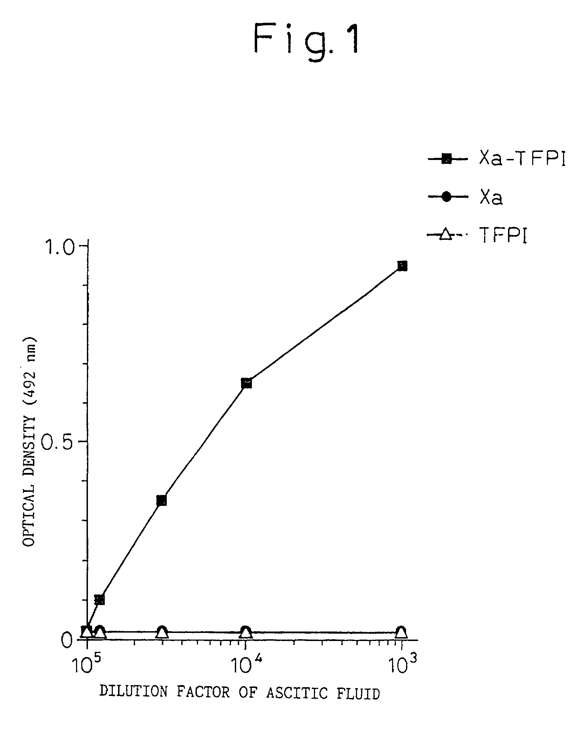

Immunoglobulin class and specificity of the anti-Xa-TFPI monoclonal antibody XT-1 were identified by the Ouchterlony immuno diffusion method and the enzyme immuno assay, respectively. The results demonstrated that the immunoglobulin class of the monoclonal antibody XT-1 was "IgG2a". The specificity was examined using a series of dilution of ascitic fluid of mice, and the results are shown in Fig. 1.

### Example 5: Latex Agglutination Immuno Assay

The anti-Xa-TFPI monoclonal antibody was immobilized to the latex (Japan Synthetic Rubber Co., Ltd., 0.497 µm) as follows:

To 10 ml of solution containing 0.1 mg/ml of the monoclonal antibody XT-1, was added the latex to a latex concentration of 1% (w/v), and the mixture was stirred vigorously at 25°C for one hour. 0.2 ml of bovine albumin solution (10 mg/ml) was added to the mixture, which was stirred vigorously at 25°C for 30 minutes and then centrifuged (20,000 xg, 30 minutes) for one hour. The residue was suspended in 50 ml of water to give a solution of the latex to which the anti-Xa-TFPI monoclonal antibody was immobilized.

The same procedure was repeated to give a solution of the latex to which the anti-TFPI monoclonal antibody was immobilized, except that anti-TFPI monoclonal antibody [the monoclonal antibody derived from the hybridoma HTFPI-K9 (deposit No. FERM-P-14467)] was used instead of the above anti-Xa-TFPI monoclonal antibody. They were used in the following latex agglutination assay.

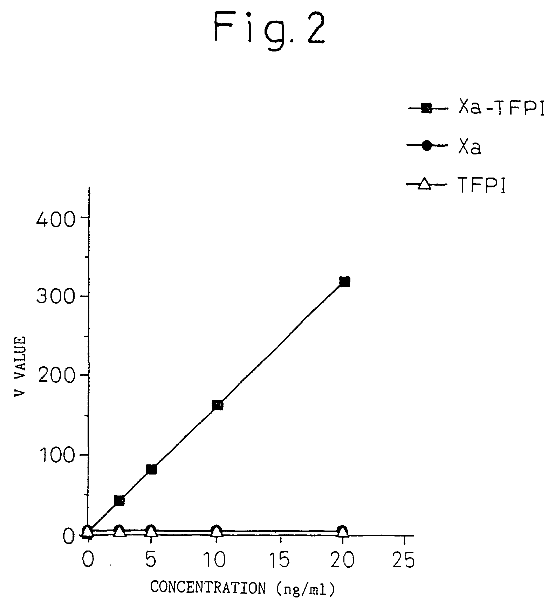

The two solutions of the latex were mixed in equivalent volumes, the mixture of the latices was mixed with sample containing Xa-TFPI to allow to react, and the velocities of agglutination were measured spectrometrically in LPIA-100 (Trade name, Mitsubishi Chemical Co., Ltd.). Because Xa-TFPI having two epitopes agglutinates, but X and TFPI having only one epitope, respectively, do not agglutinate, Xa-TFPI in the sample could be measured with accuracy. The relation between Xa-TFPI concentrations and the velocities of the agglutination reactions (the calibration curve) is shown in Fig. 2.

### Example 6: One Step Sandwich Enzyme Immuno Assay

Horseradish peroxidase was bound to the anti-Xa-TFPI monoclonal antibody XT-1 in the similar method to that by Nakane and Kawaoi [Journal of Histochemistry and Cytochemistry, vol. 22, p. 1084 - 1091 (1974)]. The sandwich enzyme immuno assay on Xa-TFPI using the enzyme-labeled antibody was as follows:

100 µl of a solution containing 10 µg/ml of the anti-TFPI monoclonal antibody (the monoclonal antibody in Example 5) in 20 mM carbonate buffer was dispensed in each of the wells in a 96-well flat bottom-type polystyrene microplate, and allowed to stand at 25°C for 30 minutes. The plate was washed 3 times with 0.05% of Tween 20 in saline. To the wells in the plate, to which the antibody was immobilized, were added 50 µl of sample, the peroxidase-labeled anti-Xa-TFPI monoclonal antibody, 0.15M MaCl, and 100 µl of 20 mM phosphate buffer containing 2% bovine serum albumin (pH 8.0).

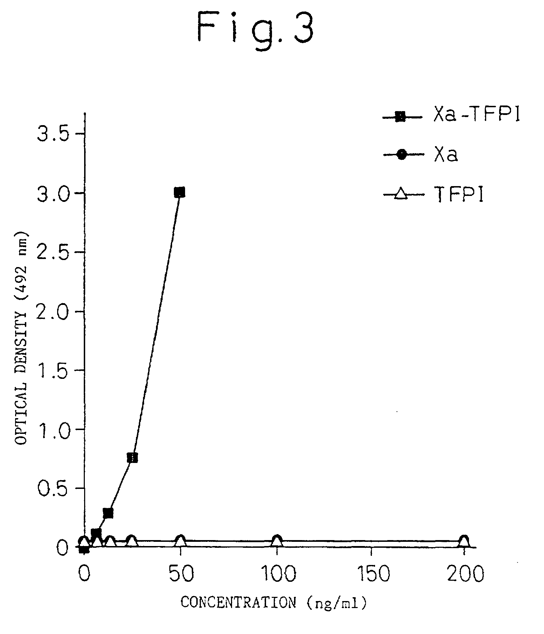

The wells were allowed to react at 25°C for 30 minutes, the plate was washed 3 times with 0.05% Tween 20 in saline. Then, 200 µl of the solution containing the enzymal substrate, which contains 10 mM phenol, 20 mM 4-aminoantipyrine and 0.005% hydrogen peroxide, was added to each of the wells. The wells were allowed to react at 25°C for 30 minutes, and the optical density at 490 nm of each of the wells was determined in a MP-590-type micro ELISA mini-reader (Dynatech Co., Ltd.). The amounts of Xa-TFPI were calculated by means of a calibration curve which was drawn using the optical densities at 490 nm, which were concurrently measured on the calibration complexes. The calibration curve is shown in Fig. 3.

### INDUSTRIAL APPLICABILITY

According to the present invention, the novel anti-Xa-TFPI monoclonal antibody which does not react with free TFPI, free Xa, and free X but reacts with Xa-TFPI with specificity, is provided, and by using the antibody, Xa-TFPI can be quantified with accuracy, as mentioned above.

Reference to Deposited Microorganism under the Rule 13-bis for the Japanese Patent Law and Depositary Authority

Depositary Authority: the National Institute of Bioscience and Human-technology (NIBH), Agency of Industrial Science and Technology

Address: 1 - 3, Higashi 1 chome, Tsukuba-shi, Ibaraki-ken, Japan

Deposit No. and Deposit Date: FERM BP-5776; December 20, 1995

## Claims

1. A monoclonal antibody which reacts with a factor Xa-tissue factor pathway inhibitor complex (Xa-TFPI) with specificity, but does not react with free factor X, free factor Xa and free tissue factor pathway inhibitor (TFPI).

2. The monoclonal antibody according to claim 1, wherein it is produced by a hybridoma XT-1 deposited as FERM BP-5776.

3. A hybridoma or the cell strain derived from it, wherein it is produced by the cell fusion of a spleen cell of a mammal immunized with Xa-TFPI with a myeloma cell, and it secrets a monoclonal antibody which reacts with Xa-TFPI with specificity, but does not react with free factor X, free factor Xa and free TFPI.

4. The hybridoma or the cell strain derived from it according to claim 3, wherein it is a hybridoma XT-1 deposited as FERM BP-5776.

5. A method for immunologically measuring Xa-TFPI, comprising using the monoclonal antibody according to claim 1 or claim 2.

## Patentansprüche

1. Ein monoklonaler Antikörper, der mit einem Faktor Xa-Gewebefaktor-Inhibitorkomplex (Xa-TFPI) spezifisch reagiert, aber nicht mit freiem Faktor X, freiem Faktor Xa und freiem Gewebefaktor-Inhibitor (TFPI) reagiert.

2. Monoklonaler Antikörper nach Anspruch 1, der von einem Hybridom XT-1, hinterlegt als FERM BP-5776, erzeugt wird.

3. Ein Hybridom oder die davon abgeleitete Zelllinie, hergestellt durch Zellfusion einer Milzzelle eines mit Xa-TFPI-immunisierten Säugers mit einer Myelomzelle, das einen spezifisch mit Xa-TFPI, aber nicht mit freiem Faktor X, freiem Faktor Xa und freiem Faktor TFPI reagierenden monoklonalen Antikörper, sezerniert.

4. Das Hybridom oder die davon abgeleitete Zelllinie nach Anspruch 3, worin das Hybridom das Hybridom XT-1 ist, das als FERM BP-5776 hinterlegt wurde.

5. Ein Verfahren zur immunologischen Bestimmung von Xa-TFPI, das den Einsatz des monoklonalen Antikörpers nach Anspruch 1 oder Anspruch 2 umfasst.

## Revendications

1. Anticorps monoclonal qui réagit de manière spécifique avec un complexe facteur Xa-inhibiteur de la voie des facteurs tissulaires (Xa-TFPI), mais qui ne réagit pas avec le facteur X libre, le facteur Xa libre et l'inhibiteur de la voie des facteurs tissulaires (Tissue Factor Pathway Inhibitor, TFPI) libre.

2. Anticorps monoclonal selon la revendication 1, lequel anticorps monoclonal est produit par un hybridome XT-1 déposé sous la référence FERM BP-5776.

3. Hybridome ou souche de cellules en dérivant, lequel hybridome est produit par la fusion cellulaire d'une cellule splénique d'un mammifère immunisé avec le complexe Xa-TFPI et d'une cellule myélomateuse, et lequel hybridome sécrète un anticorps monoclonal qui réagit de manière spécifique avec le complexe Xa-TFPI mais ne réagit pas avec le facteur X libre, le facteur Xa libre et le TFPI libre.

4. Hybridome ou souche de cellules en dérivant selon la revendication 3, lequel hybridome est un hybridome XT-1 déposé sous la référence FERM BP-5776.

5. Procédé immunologique pour mesurer le complexe Xa-TFPI comprenant l'utilisation d'un anticorps monoclonal selon la revendication 1 ou la revendication 2.
